# EUROPÄISCHE PATENTSCHRIFT

(11) **EP 0 396 879 B1**
(45) Veröffentlichungstag und Bekanntmachung des Hinweises auf die Patenterteilung: **21.12.1994**
(21) Anmeldenummer: 90105047.6
(22) Anmeldetag: 17.03.1990
(51) Int. Cl.: A61M 5/00, A61L 29/00

(54) **Übertragungssysteme für Infusions- oder Transfusionszwecke**
Transmission systems for infusion or transfusion
Systèmes de transmission pour les infusions et transfusions

(30) Priorität: 08.05.1989 DE 3914998
(43) Veröffentlichungstag der Anmeldung: 14.11.1990
(73) Patentinhaber: CLINICO INFUSIONSTECHNIK GmbH, D-36222 Bad Hersfeld (DE)
(72) Erfinder: Heinzerling, Walter C., D-6430 Bad Hersfeld (DE); Heinzerling, Jörg, Dipl.-Ing., D-6430 Bad Hersfeld (DE)
(74) Vertreter: von Raffay, Vincenz, Dipl.-Ing.

(56) Entgegenhaltungen:
- EP-A- 0 007 601
- DE-U- 8 810 468

## Beschreibung

Die Erfindung betrifft ein Übertragungssystem für Infusions- oder Transfusionszwecke nach dem Oberbegriff des Hauptanspruchs.

Übertragungssysteme bzw. -geräte und für Sonderanwendungen entwickelte Zubehörartikel für Infusions- und Transfusionstherapie sind seit Jahrzehnten in den unterschiedlichsten Ausführungen bekannt.

Diese durchweg aus thermoplastischen Kunststoffen oder elastischen Kautschuken hergestellte Systeme oder Bestandteile dieser sind notwendigerweise als Einmalartikel konzipiert, um Infektionsgefährdungen bei Mehrfachbenutzungen auszuschließen. Eine großtechnische Wiederverwertung der bislang verlorenen Rohstoffe ist nach dem Stand der Technik ausgeschlossen, da bedingt durch sehr unterschiedliche Anforderungen an die einzelnen Systemkomponenten Kunststoffe aus unterschiedlichsten Kunststoffgruppen Verwendung finden.

Einen gewissen Überblick über den Stand der Technik gibt die EP-PS 7601, in der folgende Kunststoffe erwähnt werden: Polyvinylchlorid, Polyamid, Polystyrol, Polypropylen, Styrol-Butadien-Styrol, Styrol-Isopren-Styrol, Styrol-Acrylnitril, Acrylnitril-Butadien-Styrol, Styrol-Butadien, Methylmetacrylat-Butadien-Styrol, Methylacrylat-Styrol-Acrylnitril, Polyäthylen-Äthylen-Vinylacetat, Polybuten, Polymethylpenten sowie Polyacrylsäureester, etc..
Dort wird auch schon die Verwendung von Polystyrolhomo- und Copolymerisaten für flexible Tropfkammern für Übertragungsgeräte beschrieben.
Die Liste dieser Vielzahl verschiedener Kunststoffe ließe sich beispielsweise noch erweitern durch Hinweis auf die DE-OS 22 16 987, in der noch zusätzlich Polyolefine, Polycarbonate, Zelluloseester neben PVC erwähnt werden.

Bedingt durch die Vielzahl der verwendeten Kunststoffe ist auch eine Entsorgung durch Verbrennung problematisch, da üblicherweise die Vielzahl der notwendigen Filtersysteme zur Reinigung der sehr umweltschädlichen Abgase nicht zur Verfügung stehen. Besonders hervorzuheben ist Polyvinylchlorid, dessen HCl-Gas enthaltende Verbrennungsabgase gefährlich sind. Dieser zumeist im Gewichtsanteil überwiegende Werkstoff zeigt zudem noch in bezug auf Weichmachermigration (DEHP) und Monomerabscheidungen während des Gebrauchs der Übertragungsgeräte ein sehr problematisches Verhalten. Weiter ist eine direkte Nutzung von Produktionsabfällen bedingt durch die Nichtmischbarkeit der verschiedensten Kunststoffsorten nur bedingt möglich.

Der Erfindung liegt deshalb die Aufgabe zugrunde, Übertragungs-Systeme nach dem Oberbegriff des Anspruchs 1 derart zu verbessern, daß sie nach dem Gebrauch eine problemlose Entsorgung ermöglichen und eine erhöhte Patientensicherheit bieten sowie in technischer und wirtschaftlicher Hinsicht herstellbar sind.

Diese Aufgabe wird durch in Anspruch 1 gekennzeichnete Übertragungssysteme gelöst.

Die Erfindung ermöglicht also die Herstellung von Übertragungssystemen, die umweltverträglich wiederverwertet werden können unter Verzicht auf weichgemachte Polyvinylchloride durch die Anwendung von Polystyrolpolymerisaten, -Copolymeren oder -Blockpolymeren für fast sämtliche Systembauteile, nämlich die Bestandteile a-h. Der unterschiedlich modifizierte Werkstoff wird den an die einzelnen Bauteile gestellten Anforderungen gerecht und ermöglicht bei einer Regranulierung und Reinigung des Gesamtproduktes erstmals eine Weiterverarbeitbarkeit des sortenreinen Regranulates. Weiter ist eine sinnvolle Verwertung von Produktionsabfällen wichtiger Systembauteile möglich, indem diese durch die Eigenschaft der vollständigen Mischbarkeit zu sekundären Bauteilen, die nur Schutz- oder Verpackungsfunktionen erfüllen, weiterverarbeitet werden.

Diese Vorteile reduzieren entscheidend die nicht verwertbaren Abfallmaterialien und führen damit zu einer Reduzierung der Umweltbelastung und Senkung der Herstellkosten für das Ausgangsprodukt.

Die Erfindung geht ferner davon aus, daß die jeweils gewünschten Eigenschaften der einzelnen Bestandteile durch Modifizierung des Molgewichtes, der Mengenverhältnisse bei Copolymeren oder der Taktizität bei den Blockpolymeren sowie durch Beimischung von Hilfsstoffen gesteuert werden. Hierdurch ist es nämlich möglich, folgende Funktions- bzw. Herstellungskriterien für die Übertragungsgeräte zu erfüllen:
1. Hohe Transparenz des gesamten Überleitgerätes, z.B. zur besseren Konstrolle der Tropfkammerfüllung (Luftfallenwirkung), Einstellmöglichkeit der Tropffolge als Kriterium der Flüssigkeitsmenge pro Zeiteinheit.
2. Sterilisierbarkeit (Be- und Entgasungsfreudigkeit durch hohe Gasdurchlässigkeit).
3. Flexiblität und Rückstellvermögen sowie des Tropfkammer Unterteils als auch des Schlauches.
4. Beständigkeit gegen energiereiche Strahlen ab 2,5 MRAD.
5. Integrierte Anordnung von Schlauchansatz und Einstechdorn am Tropfkammer-, Unter- bzw. Oberteil.
6. Verschweiß-/Verklebbarkeit und Umspritzung für Tropfkammer-Ober-/Unterteil bzw. aller Schlauchverbindungen mit Kegelverbindungsprinzip zur Erzielung einer flüssigkeits- und luftdichten Verbindungsstelle.
7. Hohe Schlagzähigkeit der mechanisch beanspruchten Bauteile (z.B. durch Druckbeaufschlagung).

Ein bevorzugter Kunststoff für ein erfindungsgemäßes übertragungsverfahren ist im Unteranspruch 4 angegeben.

Ferner dürfte einleuchten, daß die Erfindung die Wiederverwendung von Regeneraten möglich ist, die naturgemäß zur Reduzierung von nicht wieder verwertbaren Abfallmaterialien und damit zur optimierten Lösung von Umweltproblemen führt und zudem noch die Herstellungskosten für das Ausgangsprodukt reduziert. Ein pyrolythisches Zerlegen des regranulierten gebrauchten Produktes ist aufgrund der relativen Homogenität des Werkstoffes weitaus günstiger.

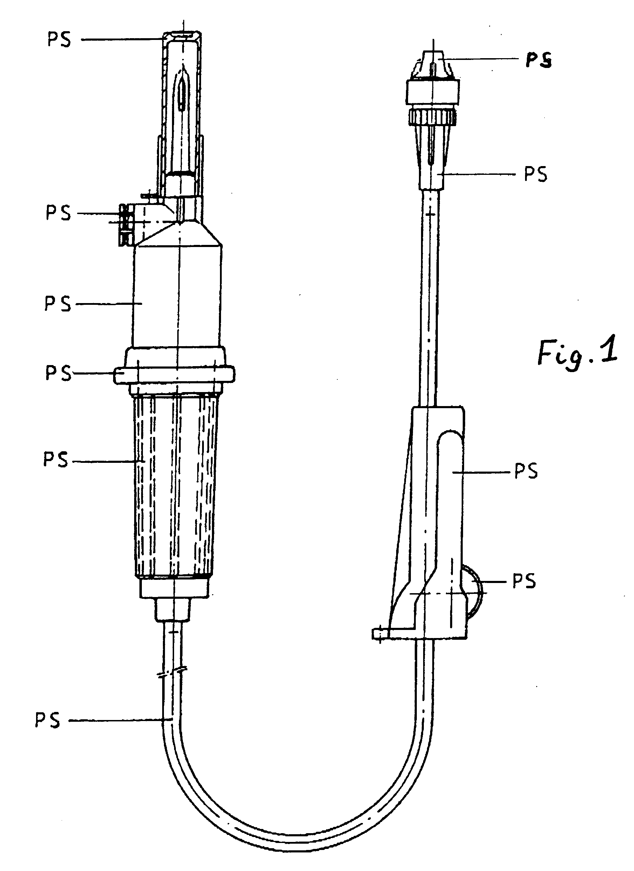

Im folgenden wird ein bevorzugtes Ausführungsbeispiel anhand der Zeichnung näher erläutert.

Es zeigt: eine schematische Querschnittsansicht durch ein erfindungsgemäßes Übertragungsgerät.

Das erfindungsgemäße Übertragungsgerät eignet sich für verschiedene Infusions- bzw. Transfusionsvorgänge. Seine sämtlichen Einzelbestandteile bestehen aus dem gleichen Grundwerkstoff, nämlich aus Polystyrol-Polymerisat. Hierdurch bedingt liegt nur noch ein leicht ökologisch recyklisierbarer Werkstoff vor und keine Vielzahl von Werkstoffkombinationen, die üblicherweise bei der Abfallentsorgung anfallen. Besonders zu erwähnen ist hierbei, daß eine problemlose Verbrennung von Polystyrolpolymerisaten im Vergleich zum bisherigen PVC-Material (keine Entstehung von HCl-Verbrennungsgasen) möglich ist.

Die Wiederverwendung des recyklisierten Kunststoffproduktes ist ebenfalls leicht möglich, da keine undefinierbaren Kunststoffgemische anfallen.

Von besonderer Bedeutung ist ebenfalls die Tatsache, daß die für den Einzelbestandteil jeweils gewünschten Eigenschaften dadurch erzeugt werden können, daß das Molgewicht des Polystyrol-Polymerisats entsprechend eingestellt wird. Es dürfte einleuchten, daß bei Co- und Blockpolymeren auf die Eigenschaften noch Einfluß auf die Mengenverhältnisse und die Taktizität genommen werden kann. Letzteres ist natürlich auch über sonst üblicher Zusätze zu erreichen.

## Patentansprüche

1. Übertragungssystem für Infusions- oder Transfusionszwecke, umfassend folgende Bestandteile aus Kunststoff:
a. Tropfkammer mit Ober- und Unterteil
b. Verbindungsstück für das Ober- und Unterteil
c. Einstechdorn
d. Schutzkappe für den Einstechdorn und das Anschlußstück, sowie das Anschlußstück selbst;
e. Belüftungsverschluß
f. Übertragungsschlauch (-schläuche)
g. Durchflußregler
h. Schlauchverbindungsstück(e)
dadurch gekennzeichnet,
daß zur umweltverträglichen Recyklisierung sämtliche Bestandteile aus Polystyrolpolymeren, -Copolymeren oder -Blockpolymeren unter Verzicht auf Polyvinylchlorid bestehen.

2. Übertragungssystem nach Anspruch 1, dadurch gekennzeichnet, daß ferner folgende Bestandteile aus Polystyrolpolymeren, -Copolymeren oder -Blockpolymeren unter Verzicht auf Polyvinylchlorid bestehen:
i. Verbindungs- und Verlängerungsstücke
j. Absperrhähne
k. Entnahme-/Übertragungskanülen

3. Übertragungssystem nach Anspruch 1 und 2,
dadurch gekennzeichnet, daß die jeweils gewünschten Eigenschaften der einzelnen Bestandteile durch Modifizierung des Molgewichtes, der Mengenverhältnisse oder Taktizität gesteuert werden.

4. Übertragungssystem nach Anspruch 1, 2 und 3,
dadurch gekennzeichnet, daß ausschließlich Polystyrolhomopolymerisat verwendet wird.

## Claims

1. Transfer system for infusion or transfusion purposes comprising the following plastic components:
a. a dropping chamber with an upper and lower part,
b. a connecting piece for the upper and lower part,
c. a perforating pin,
d. a protective cap for the perforating pin and the connecting piece, as well as the actual connecting piece,
e. a vent cap,
f. a transfer hose or hoses,
g. a flow regulator,
h. a hose connecting piece or pieces,
characterized in that
for environmentally compatible recycling all the components are made from polystyrene polymers, copolymers or block copolymers, without using polyvinyl chloride.

2. Transfer system according to claim 1, characterized in that in addition the following components are made from polystyrene polymers, copolymers or block polymers, whilst not using polyvinyl chloride:
i. connection and extension pieces,
j. stopcocks,
k. withdrawal/transfer cannulas.

3. Transfer system according to claim 1 and 2, characterized in that the in each case desired characteristics of the individual components are controlled by modifying the molecular weight, quantity ratio or tacticity.

4. Transfer system according to claim 1,2 and 3, characterized in that exclusively polystyrene homopolymer is used.

## Revendications

1. Système à perfusion pour usage en perfusions et transfusions, comprenant les composantes en matière synthétique suivantes :
a. chambre compte-gouttes composée d'une partie supérieure et d'une partie inférieure
b. raccord pour la partie supérieure et la partie inférieure
c. perforateur
d. capuchon de protection du perforateur et de l'embout, ainsi que l'embout lui-même;
e. sortie d'air
f. tubulure à perfusion (tubulures)
g. régulateur de débit
h. raccord(s) de la tubulure
caractérisé par le fait,
que toutes les composantes soient en polymères, copolymères et polymères en masse de polystyrène, sans addition de PVC, pour un recyclage écophile.

2. Système à perfusion selon revendication 1, caractérisé par le fait, que ces autres composantes soient en polymères, copolymères et polymères en masse de polystyrène, sans addition de PVC :
i. raccords et rallonges
j. robinets d'arrêt
k. canules de prélèvement/à perfusion

3. Système à perfusion selon revendications 1 et 2, caractérisé par le fait, que les propriétés souhaitées des différentes composantes soient commandées par modification du poids moléculaire, des rapports volumétriques ou du tactisme.

4. Système à perfusion selon revendications 1, 2 et 3, caractérisé par le fait que seul un homopolymère de polystyrène soit utilisé.
